# EUROPÄISCHE PATENTANMELDUNG

(11) **EP 1 977 775 A1**
(43) Veröffentlichungstag der Anmeldung: **08.10.2008**
(21) Anmeldenummer: 08103389.6
(22) Anmeldetag: 04.04.2008
(51) Int. Cl.: A61M 5/14, A61M 5/168, F15C 5/00, F16K 99/00, A61M 5/142

(54) **Infusionspumpe**

(30) Priorität: 04.04.2007 DE 102007016659
(71) Anmelder: Tricumed Medizintechnik GmbH, 24143 Kiel (DE)
(72) Erfinder: Otto, Karl-Heinz, 24143, Kiel (DE)
(74) Vertreter: Schäfer, Horst

(57) **Zusammenfassung**

Eine implantierbare Infusionspumpe weist ein Gehäuse (12) und einen innerhalb des Gehäuses (12) angeordneten Medikamentenraum (11) zur Aufnahme eines Medikaments sowie eine die Abgabe des Medikamentes dosierenden, zu einem im Körper des Patienten mündenden Katheter führende Drosselstrecke auf. Die Drosselstrecke ist als in eine Oberseite (2) einer Kanalplatte (1) eingebrachter Kanal (4) ausgebildet. Bereiche (27) der Kanalplatte (1) weisen ein mikrostrukturierbares Material auf und Oberflächen (25, 26) des Kanals (4), die mit dem Medikament in Kontakt treten, weisen ein gegenüber basischen Lösungen mit einem pH-Wert von pH ≥ 10 resistentes Material auf.

## Beschreibung

Die Erfindung betrifft eine in den Körper eines Patienten implantierbare Infusionspumpe, eine Kanalplatte für die Infusionspumpe und ein Verfahren zur Herstellung der Infusionspumpe.

Implantierbare Infusionspumpen weisen ein Gehäuse auf, in dem ein Medikamentenraum zur Aufnahme und Vorhaltung eines Medikaments, beispielsweise eines Schmerzmittels, angeordnet ist. Bei Infusionspumpen, die besonders einfach aufgebaut sind und keine elektronische Regelung der Flussrate aufweisen, erfolgt die Abgabe des Medikaments über eine zu einem im Körper des Patienten mündenden Katheter führende Drosselstrecke, wobei die Dosierung des Medikaments über die Geometrie, insbesondere über Länge und Durchmesser, der Drosselstrecke eingestellt wird.

Eine solche Infusionspumpe ist beispielsweise aus der EP 0 189 940 A2 bekannt. Sie weist eine Drosselstrecke auf, die als Kanal in einen Siliziumchip geätzt wurde. Dies hat den Vorteil, dass aus der Halbleitertechnologie bekannte und bewährte Verfahren der Photolithographie und Ätztechnik zum Einsatz kommen und sehr feine Strukturen hergestellt werden können. Auf diese Weise lassen sich Drosselstrecken mit einer großen Länge herstellen, was nach dem Gesetz von Hagen-Poiseuille einem geringen Volumendurchsatz entspricht.

Allerdings sind die sehr feinen Strukturen besonders anfällig für eine Beschädigung beispielsweise durch Verschleiß, wie er bei einer dauerhaften hohen Beanspruchung der Drosselstrecke auftritt. Beschädigungen der die Drosselstrecke bildenden Strukturen können zu drastischen Veränderungen der Fördermenge der Infusionspumpe führen und somit eine Gefahr für den Patienten darstellen.

Aufgabe der Erfindung ist es daher, implantierbare Infusionspumpen im Hinblick auf eine lange Lebensdauer und einen sicheren Einsatz weiter zu verbessern. Eine weitere Aufgabe ist es, ein besonders einfaches Verfahren zur Herstellung implantierbarer Infusionspumpen anzugeben.

Erfindungsgemäß wird diese Aufgabe mit dem Gegenstand der unabhängigen Patentansprüche gelöst. Vorteilhafte Weiterbildungen der Erfindung sind Gegenstand der abhängigen Patentansprüche.

Eine erfindungsgemäße Infusionspumpe weist ein Gehäuse und einen innerhalb des Gehäuses angeordneten Medikamentenraum zur Aufnahme eines Medikaments auf. Sie weist ferner eine die Abgabe des Medikamentes dosierende, zu einem im Körper des Patienten mündenden Katheter führende Drosselstrecke auf, wobei die Drosselstrecke als in eine Oberseite einer Kanalplatte eingebrachter Kanal ausgebildet ist. Bereiche der Kanalplatte weisen ein mikrostrukturierbares Material auf und Oberflächen des Kanals, die mit dem Medikament in Kontakt treten, weisen ein gegenüber basischen Lösungen mit einem pH-Wert von pH ≥ 10 resistentes Material auf.

Wie sich nämlich herausgestellt hat, sind Siliziumchips nicht ausreichend beständig gegenüber basischen Lösungen mit pH-Werten von ca. 10 und darüber. In der Medizin eingesetzte Trägerflüssigkeiten wie beispielsweise NaCl können jedoch insbesondere einen solchen hohen pH-Wert aufweisen. Einem Grundgedanken der Erfindung zufolge sollte daher für die Bereiche bzw. Oberflächen der Kanalplatte, die mit dem Medikament in Kontakt kommen, ein gegenüber basischen Lösungen möglichst beständiges Material verwendet werden. Andererseits sollte die Herstellung besonders feiner Strukturen möglich sein. Letzteres kann durch die Verwendung von aus der Halbleitertechnologie bekannten Verfahren erreicht werden.

Unter einem gegenüber basischen Lösungen resistenten Material wird somit hier und im folgenden ein Material verstanden, das gegenüber in der Medizin angewandten Trägerflüssigkeiten mit einem pH-Wert von pH ≥ 10 derart beständig bzw. inert ist, dass eine Drosselstrecke bildende Kanalstrukturen während einer typischerweise angestrebten Lebensdauer einer implantierbaren Infusionspumpe von einigen Jahren nicht in einem Maße angegriffen werden, dass die Funktion der Infusionspumpe beeinträchtigt wird.

In einer Ausführungsform der Erfindung ist als mikrostrukturierbares Material ein fotoempfindliches Glas vorgesehen. Dieses ist sehr einfach mit photolithografischen Methoden strukturierbar. Es weist jedoch zusätzlich auch eine hohe Beständigkeit gegenüber basischen Lösungen auf, so dass es auch als gegenüber basischen Lösungen resistentes Material vorgesehen sein kann. In einer Ausführungsform der Erfindung ist die Kanalplatte vollständig aus fotoempfindlichem Glas ausgebildet. In diesem Fall ist das gesamte Material der Kanalplatte sowohl mikrostrukturierbar als auch resistent gegenüber Basen.

Unter einem fotoempfindlichen Glas wird hier und im folgenden ein Glas wie beispielsweise Foturan verstanden, das durch Belichtung mit Photonen einer bestimmten Wellenlänge seine Struktur verändert und in den belichteten Bereichen Nanokristalle oder andere Kristallisationskeime ausbildet. Diese Kristallisationskeime können durch Wärmezufuhr zu einer Kristallstruktur wachsen, die für ein Ätzmittel wesentlich leichter angreifbar ist als die noch amorphen Bereiche des Glases. Folglich können die kristallinen Bereiche in einem Ätzprozess leicht entfernt werden. Solche Gläser werden auch "mikrostrukturierbar" oder "fotostrukturierbar" genannt.

In einer alternativen Ausführungsform ist als mikrostrukturierbares Material Silizium oder eine Siliziumverbindung vorgesehen. Silizium hat als Standardmaterial in der Halbleitertechnologie den Vorteil, dass es preiswert ist und mit bewährten Verfahren verarbeitet werden kann. Um es vor dem Einfluss basischer Lösungen zu schützen, ist bei dieser Ausführungsform zumindest für alle Oberflächen der Kanalplatte, die mit dem Medikament in Kontakt kommen, ein anderes, gegenüber basischen Lösungen resistentes Material vorgesehen.

Dieses Material kann als Beschichtung auf die Kontaktbereiche des bereits ausgebildeten Kanals aufgebracht werden, es muss somit nicht selbst mikrostrukturierbar sein. In einer Ausführungsform ist als gegenüber basischen Lösungen resistentes Material Glas vorgesehen. Dabei kommen wegen ihrer guten chemischen Beständigkeit beispielsweise Borsilikatglas, Quarzglas oder Kalknatronglas in Frage.

In einer alternativen Ausführungsform ist als gegenüber basischen Lösungen resistentes Material ein Kunststoff vorgesehen. Kunststoffe können sehr einfach durch physikalische oder chemische Gasphasenabscheidung oder andere gängige Beschichtungsverfahren auch auf nicht so leicht zugängliche Oberflächen aufgebracht werden. In einer Ausführungsform ist zur Beschichtung PTFE vorgesehen. Alternativ kann als gegenüber basischen Lösungen resistentes Material DLC (diamon-like carbon) vorgesehen sein.

Auch andere Kunststoffe weisen eine sehr gute Beständigkeit gegen Basen auf. Als gegenüber basischen Lösungen resistentes Material kommen daher beispielsweise auch Kunststoffe aus der Gruppe bestehend aus Polyimid, Polyamid, Polyethylen, EVA, Polypropylen, Polymethylmethacrylat und PVC in Betracht.

In einer Ausführungsform der Erfindung weist der Kanal eine Spiralform auf. Es sind jedoch auch andere, beispielsweise mäanderförmige Strukturen denkbar, die den auf der Kanalplatte vorhandenen Platz möglichst gut zur Bildung einer langen Drosselstrecke ausnutzen. Die Spiralform des Kanals hat den Vorteil, dass ein besonders gleichmäßiger Fluss des Medikaments durch die Drosselstrecke möglich ist. Bei mäandrisch verlaufenden Drosselstrecken besteht dagegen eher die Gefahr, dass, insbesondere bei langen Molekülen, im Totwassergebiet der Ecken Bestandteile des Medikaments auskristallisieren oder sich ablagern.

Der Querschnitt des Kanals ist in einer Ausführungsform der Erfindung im Wesentlichen rechteckig. Ein Kanal mit rechteckigen Querschnitt entsteht durch eine gleichmäßige Belichtung und damit eine gleichmäßige Eindringtiefe des Lichts in das Glas.

An der Oberseite der Kanalplatte liegt der die Drosselstrecke bildende Kanal frei. Um eine allseitig geschlossene Fluidbahn für das Medikament zu bilden, wird der Kanal vorteilhafterweise mit einer Deckelplatte abgedeckt. Die Infusionspumpe weist dazu eine Deckelplatte mit einer ersten Seite und einer zweiten Seite auf, wobei die erste Seite der Deckelplatte an der Oberseite der Kanalplatte anliegt.

In einer vorteilhaften Ausführungsform ist die Deckelplatte aus Glas ausgebildet. Damit ist sichergestellt, dass auch die Deckelplatte, die mit dem Medikament ebenfalls in Berührung kommt, beständig gegenüber basischen Lösungen ist. Dabei kann die Deckelplatte aus dem gleichen Glas bestehen wie die Kanalplatte, es ist jedoch auch möglich, für die Deckelplatte ein anderes, beispielsweise nicht fotoempfindliches Glas zu verwenden. Auch die Verwendung einer Deckelplatte aus Edelstahl oder Titan oder einem anderen Material ist grundsätzlich denkbar.

Bei der erfindungsgemäßen Infusionspumpe ist ein Fließweg für das Medikament durch einen Einlass in einem Verschluss des Medikamentenraums, durch einen Durchlass durch die Deckelplatte, durch einen Durchlass durch die Kanalplatte und durch den Kanal gebildet. Dazu weist der Kanal einen Beginn und ein Ende auf, wobei der Beginn des Kanals in einer Fließrichtung des Medikaments vor dem Ende des Kanals liegt.

In einer Ausführungsform der Erfindung ist die Kanalplatte mit ihrer Unterseite und ihren Randseiten in das Gehäuse eingebettet und die Deckelplatte sandwichartig zwischen der Kanalplatte und einem Verschluss des Medikamentenraums angeordnet. Der Beginn des Kanals fluchtet mit dem Durchlass durch die Deckelplatte und dem Einlass in dem Verschluss des Medikamentenraums. Das Ende des Kanals fluchtet mit dem Durchlass durch die Kanalplatte und einem Auslass in dem Gehäuse.

Bei dieser Ausführungsform kann die zweite Seite der Deckelplatte gegen den Verschluss des Medikamentenraums mittels eines O-Ringes aus einem gummielastischen Material abgedichtet sein, wobei vorteilhafterweise in dem Verschluss des Medikamentenraums Ausnehmungen zur Aufnahme des O-Rings angeordnet sind.

In einer alternativen Ausführungsform der Erfindung ist die Deckelplatte mit ihrer zweiten Seite und ihren Randseiten in das Gehäuse eingebettet und die Kanalplatte sandwichartig zwischen der Deckelplatte und einem Verschluss des Medikamentenraums angeordnet. Im Gegensatz zu der vorstehend beschriebenen Ausführungsform ist bei dieser alternativen Ausführungsform also die Reihenfolge, in der Deckelplatte, Kanalplatte und Verschluss des Medikamentenraums übereinander gestapelt sind, verändert. Dabei fluchtet der Beginn des Kanals mit dem Durchlass durch die Kanalplatte und dem Einlass in dem Verschluss des Medikamentenraums und das Ende des Kanals fluchtet mit dem Durchlass durch die Deckelplatte und einem Auslass in dem Gehäuse.

Bei dieser Ausführungsform kann die zweite Seite der Deckelplatte gegen das Gehäuse mittels eines O-Ringes aus einem gummielastischen Material abgedichtet sein, wobei vorteilhafterweise in dem Gehäuse Ausnehmungen zur Aufnahme des O-Rings angeordnet sind.

Zur Erzeugung eines das Medikament entgegen dem Fließwiderstand der Drosselstrecke austreibenden Drucks ist in einer Ausführungsform in dem Gehäuse ein Treibmittelraum zur Aufnahme eines verdampfbarem Treibmittels angeordnet.

Die erfindungsgemäße Infusionspumpe bietet ein besonders günstiges Verhältnis von Bauvolumen zu Fällvolumen und eignet sich daher besonders zur Implantation in den Körper eines Patienten,

Ein weiterer Aspekt der vorliegenden Erfindung betrifft eine Kanalplatte mit einer Drosselstrecke für eine Infusionspumpe, wobei die Drosselstrecke die Abgabe eines Medikamentes dosiert und zu einem im Körper des Patienten mündenden Katheter führt. Die Drosselstrecke ist als in eine Oberseite der Kanalplatte eingebrachter Kanal ausgebildet und Bereiche der Kanalplatte weisen ein mikrostrukturierbares Material auf. Oberflächen des Kanals, die mit dem Medikament in Kontakt treten, weisen ein gegenüber basischen Lösungen resistentes Material auf.

Der Kanal weist einen Beginn und ein Ende auf, wobei der Beginn des Kanals in Fließrichtung des Medikaments vor dem Ende des Kanals liegt. In einer Ausführungsform der Kanalplatte fluchtet der Beginn des Kanals mit einen Durchlass durch die Kanalplatte, wobei der Durchlass durch die Kanalplatte vom Boden des Kanals abzweigt. In einer weiteren Ausführungsform fluchtet das Ende des Kanals mit einen Durchlass durch die Kanalplatte.

Ein erfindungsgemäßes Verfahren zur Herstellung einer Kanalplatte mit einer Drosselstrecke für eine Infusionspumpe weist folgende Schritte auf: zunächst wird eine Kanalplatte mit einer Oberseite und einer Unterseite bereitgestellt, wobei zumindest die Oberseite fotoempfindliches Glas aufweist. Ferner wird eine Fotolithografiemaske zum Aufbringen eines Kanalmusters auf die Oberseite der Kanalplatte bereitgestellt. Die Oberseite der Kanalplatte wird durch die Fotolithografiemaske mit Licht einer Wellenlänge λ_{G}, für die das Glas fotoempfindlich ist, belichtet. Anschließend wird die Kanalplatte durch Erhitzen auf eine Temperatur T_{G} wärmebehandelt, wobei bei der Temperatur T_{G} in den belichteten Bereichen eine Umwandlung der amorphen Glasstruktur in eine keramische Kristallstruktur erfolgt. Anschließend erfolgt das Ätzen eines eine Drosselstrecke ausbildenden Kanals in die Oberseite der Kanalplatte unter Inkontaktbringen der Kanalplatte mit einer Ätzmittellösung und unter Entfernen der kristallinen Phase.

In einer alternativen Ausführungsform des Verfahrens wird das Einbringen eines Kanalmusters in die Oberseite der Kanalplatte durch direktes "Beschreiben" der Kanalplatte mit einem Laser vorgenommen. In diesem Fall ist das Aufbringen einer Fotolithografiemaske nicht erforderlich und das selektive Belichten derjenigen Bereiche der Oberseite der Kanalplatte, die zu Kanälen strukturiert werden sollen, erfolgt durch die Führung des Laserstrahls.

Das Verfahren weist den Vorteil auf, dass durch die Verwendung von Glas anstelle eines kristallinen Substrats auf eine aufwendige Eckenkompensation beim nasschemischen Ätzen verzichtet werden kann. Zudem wird das fotoempfindliche Glas direkt belichtet, das Aufbringen eines fotoempfindlichen Lacks entfällt somit ebenfalls. Mit dem erfindungsgemäßen Verfahren können besonders feine Strukturen in die Glasoberfläche eingebracht werden. Es ist daher möglich, eine besonders lange Drosselstrecke mit geringen Durchmesser und hohem Fließwiderstand herzustellen.

In einer Ausführungsform des Verfahrens erfolgt das Belichten der Oberseite der Kanalplatte durch die Fotolithografiemaske mit Licht der Wellenlänge λ_{G} mit 290≤ λ_{G} ≤ 330 nm und die Kanalplatte wird zur Wärmebehandlung auf die Temperatur 500 °C ≤ T_{G} ≤ 600 °C erhitzt.

Als Ätzmittellösung kann beispielsweise eine 10%ige Flusssäurelösung verwendet werden.

Das Ätzen selbst wird vorteilhafterweise bei Raumtemperatur durchgeführt.

Ein alternatives Verfahren zur Herstellung einer Kanalplatte mit einer Drosselstrecke für eine Infusionspumpe weist folgende Schritte auf: zunächst wird eine Kanalplatte mit einer Oberseite und einer Unterseite bereitgestellt, wobei zumindest die Oberseite Bereiche eines mikrostrukturierbaren Materials aufweist. Ferner wird eine Fotolithografiemaske zum Aufbringen eines Kanalmusters auf die Oberseite der Kanalplatte bereitgestellt. Anschließend wird ein Fotolack auf die Oberseite der Kanalplatte aufgebracht und die Oberseite der Kanalplatte wird durch die Fotolithografiemaske belichtet. Anschließend erfolgt das Ätzen eines eine Drosselstrecke ausbildenden Kanals in die Oberseite der Kanalplatte unter Inkontaktbringen der Kanalplatte mit einer Atzmittellösung. Oberflächen des Kanals, die mit einem Medikament in Kontakt treten, werden mit einem gegenüber basischen Lösungen resistenten Material beschichtet.

Das Beschichten der Oberflächen kann dabei beispielsweise durch physikalische oder chemische Gasphasenabscheidung erfolgen.

Ein erfindungsgemäßes Verfahren zur Herstellung einer Infusionspumpe weist zusätzlich folgende Verfahrensschritte auf: Nach der Herstellung der Kanalplatte wird zumindest ein Durchlass in die Kanalplatte eingebracht, der mit einem Beginn des Kanals fluchtet. Der Durchlass bildet dabei eine Fluidbahn für das Medikament durch die Dicke der Kanalplatte hindurch. Auf die Oberseite der Kanalplatte wird eine Deckelplatte aus Glas aufgebondet, wobei ein Durchlass in der Deckelplatte mit dem Ende des Kanals fluchtet. Dieser Durchlass bildet somit eine Fluidbahn für das Medikament durch die Dicke der Deckelplatte hindurch. Anschließend wird die Kanalplatte mit aufgebondeter Deckelplatte in ein Gehäuse eingesetzt.

In einer alternativen Ausführungsform des Verfahrens wird der Durchlass in die Kanalplatte derart eingebracht, dass er mit einem Ende des Kanals fluchtet. In diesem Fall weist die Deckelplatte einen Durchlass auf, der mit dem Beginn des Kanals fluchtet.

Ausführungsbeispiele der Erfindung werden im folgenden anhand der beigefügten Figuren näher erläutert.
- Figur 1: zeigt schematisch eine Kanalplatte gemäß einem Ausführungsbeispiel der Erfindung;
- Figur 2: zeigt schematisch einen ersten Schritt eines Verfahrens zur Herstellung einer Kanalplatte gemäß einem Ausführungsbeispiel der Erfindung;
- Figur 3: zeigt schematisch einen zweiten Schritt eines Verfahrens zur Herstellung einer Kanalplatte gemäß einem Ausführungsbeispiel der Erfindung;
- Figur 4: zeigt schematisch einen dritten Schritt eines Verfahrens zur Herstellung einer Kanalplatte gemäß einem Ausführungsbeispiel der Erfindung;
- Figur 5: zeigt schematisch eine Kanalplatte gemäß einem alternativen Ausführungsbeispiel der Erfindung;
- Figur 6: zeigt schematisch einen Querschnitt durch eine Infusionspumpe gemäß einem ersten Ausführungsbeispiel der Erfindung und
- Figur 7: zeigt schematisch einen Querschnitt durch eine Infusionspumpe gemäß einem zweiten Ausführungsbeispiel der Erfindung.

Gleiche Teile sind in allen Figuren mit den gleichen Bezugszeichen versehen.

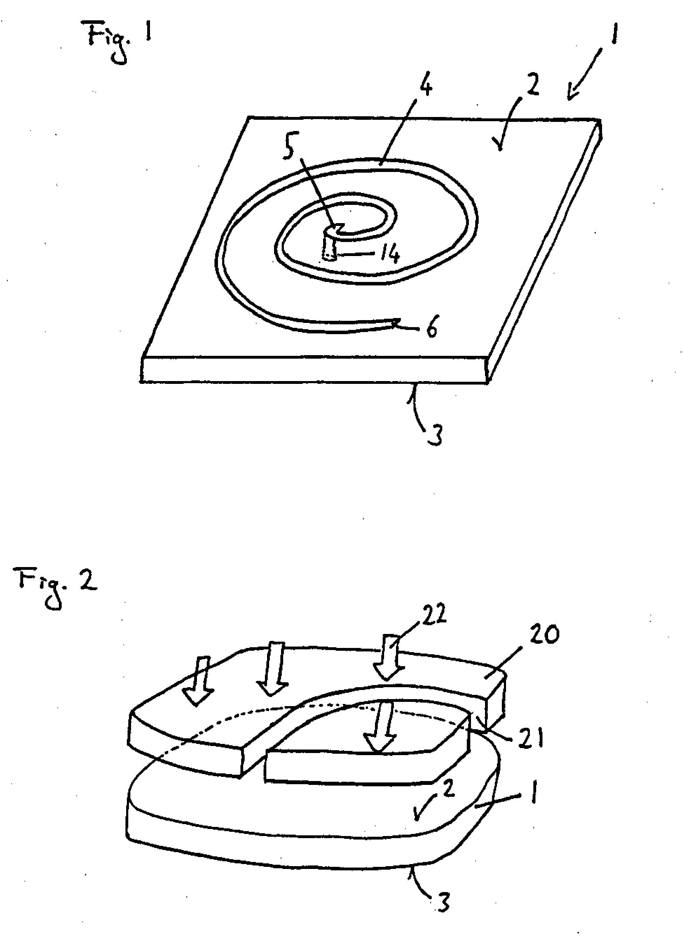

Figur 1 zeigt schematisch eine Kanalplatte 1 gemäß einem Ausführungsbeispiel der Erfindung. In diesem Ausführungsbeispiel besteht die Kanalplatte 1 vollständig aus fotoempfindlichem Glas, beispielsweise aus Foturan. Sie weist eine Oberseite 2 und eine Unterseite 3 auf. In die Oberseite 2 der Kanalplatte 1 ist ein Kanal 4 eingebracht, der in diesem Ausführungsbeispiel spiralförmig ausgebildet ist.

Der Kanal 4 ist als Vertiefung in der Oberseite 2 der Kanalplatte 1 ausgebildet und weist einen Beginn 5 und ein Ende 6 auf, wobei der Beginn 5 in Fließrichtung eines Medikaments vor dem Ende 6 des Kanals 4 angeordnet ist. In dem gezeigten Ausführungsbeispiel ist der Beginn 5 des Kanals 4 im Zentrum der Spirale angeordnet.

Die Kanalplatte 1 weist ferner einen Durchlass 14 auf, der in Figur 1 gestrichelt angedeutet ist. Der Durchlass 14 fluchtet mit dem Beginn 5 des Kanals 4 und fungiert in diesem Ausführungsbeispiel als Einlass für ein Medikament in den Kanal 4.

Der Kanal 4 gemäß Figur 1 bildet eine Drosselstrecke für eine implantierbare Infusionspumpe, durch deren geeignet gewählte Länge und Querschnitt die Abgabe eines Medikaments an den Körper eines Patienten dosiert werden kann.

Die Figuren 2 bis 4 zeigen schematisch Schritte eines Verfahrens zur Herstellung einer Kanalplatte gemäß einem Ausführungsbeispiel der Erfindung.

Figur 2 zeigt einen Ausschnitt aus der Kanalplatte 1, in die noch kein Kanal eingebracht ist. Die Oberseite 2 der Kanalplatte 1 wird durch eine Maske 20 mit UV-Strahlung, die durch die Pfeile 22 angedeutet ist, belichtet. Dazu wird eine Strahlung der Wellenlänge λ_{G} verwendet, für die das Glas der Kanalplatte 1 empfindlich ist. Die Maske 20 weist eine Struktur aus Durchbrüchen 21 auf, durch die Bereiche der Oberseite 2 der Kanalplatte 1 belichtet werden.

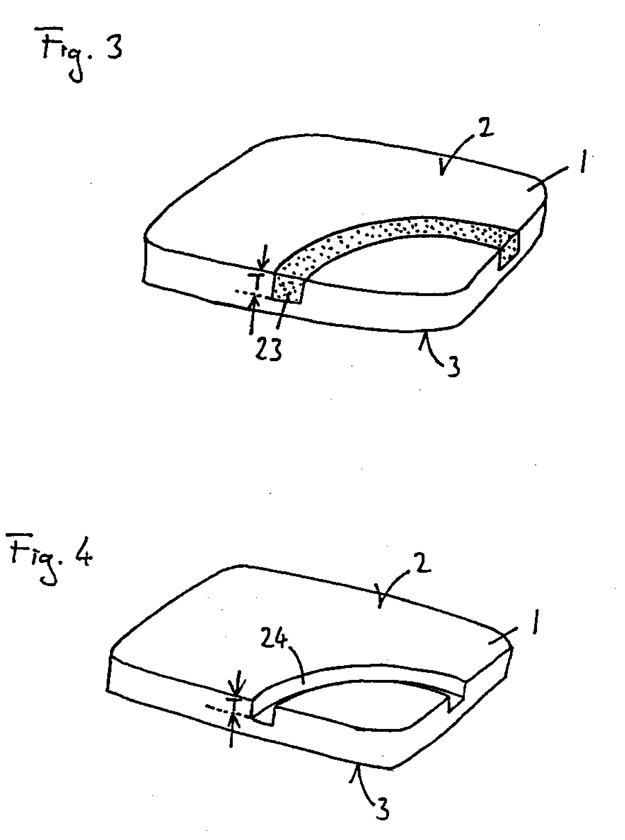

Figur 3 zeigt das Ergebnis des in Figur 2 dargestellten Verfahrensschrittes. Die Kanalplatte 1 weist in ihrer Oberseite 2 belichtete Bereiche 23 auf, die bis in eine Tiefe T in die Kanalplatte 1 hinein reichen. In den belichteten Bereichen 23 ist die Mikrostruktur des Glases verändert. In diesen Ausführungsbeispiel wurden durch die Belichtung freie Silberatome gebildet, die als Kristallisationskeime dienen. In einem an die Belichtung anschließenden Temperschritt beispielsweise bei einer Temperatur zwischen 500 und 600 °C bildet sich daher in den belichteten Bereichen 23 eine Kristallstruktur in dem ansonsten amorphen Glas der Kanalplatte 1 aus.

An das Tempern schließt sich ein hier nicht dargestellter Ätzschritt an. Aufgrund ihrer kristallinen Struktur weisen die belichteten Bereiche 23 eine bis zu etwa 20fach erhöhte Ätzrate gegenüber den unbelichteten Bereichen auf. Sie werden daher durch ein Ätzmittel wie beispielsweise Flusssäure verstärkt angegriffen. Mit diesem Verfahren lassen sich somit Strukturen mit einem hohen Aspektverhältnis (Verhältnis zwischen Tiefe und Breite der Strukturen) erzielen, wobei das Aspektverhältnis des Kanals in den gezeigten Figuren nicht unbedingt realistisch dargestellt ist.

Figur 4 zeigt das Ergebnis des Ätzens. In die Oberseite 2 der Kanalplatte 1 ist eine Grabenstruktur 24 mit einer Tiefe T eingebracht worden, die einen Abschnitt des Kanals bildet.

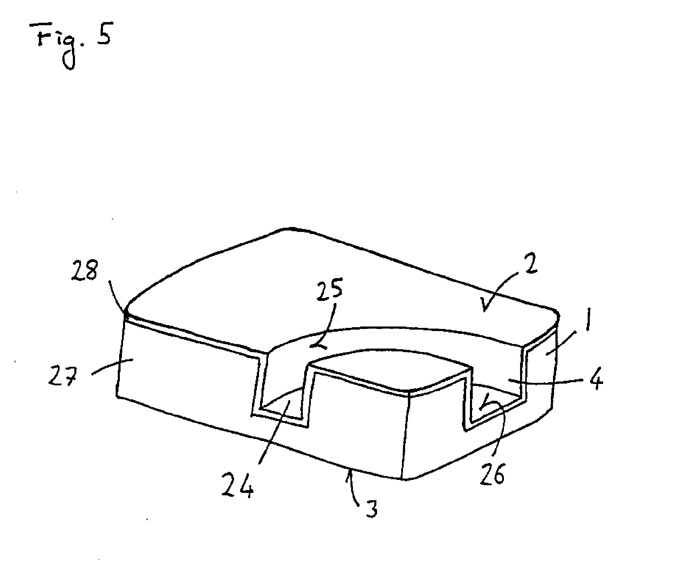

Figur 5 zeigt schematisch eine Kanalplatte 1 gemäß einem alternativen Ausführungsbeispiel der Erfindung. In diesem Ausführungsbeispiel besteht die Kanalplatte 1 nicht aus fotoempfindlichem Glas, sondern sie weist Bereiche 27 aus einem mikrostrukturierbaren Material und Bereiche 28 aus einem gegenüber basischen Lösungen mit einem pH-Wert von größer oder gleich 10 resistenten Material auf, wobei die beiden Materialien unterschiedlich sind. In diesem Ausführungsbeispiel ist die gesamte Oberseite 2 der Kanalplatte einschließlich der Wände 25 und des Bodens 26 des Kanals 4 mit dem gegenüber basischen Lösungen resistenten Material beschichtet. Es ist jedoch auch möglich, lediglich diejenigen Oberflächen zu beschichten, die mit dem Medikament in Berührung kommen, also die Wände 25 und den Boden 26 des Kanals 4.

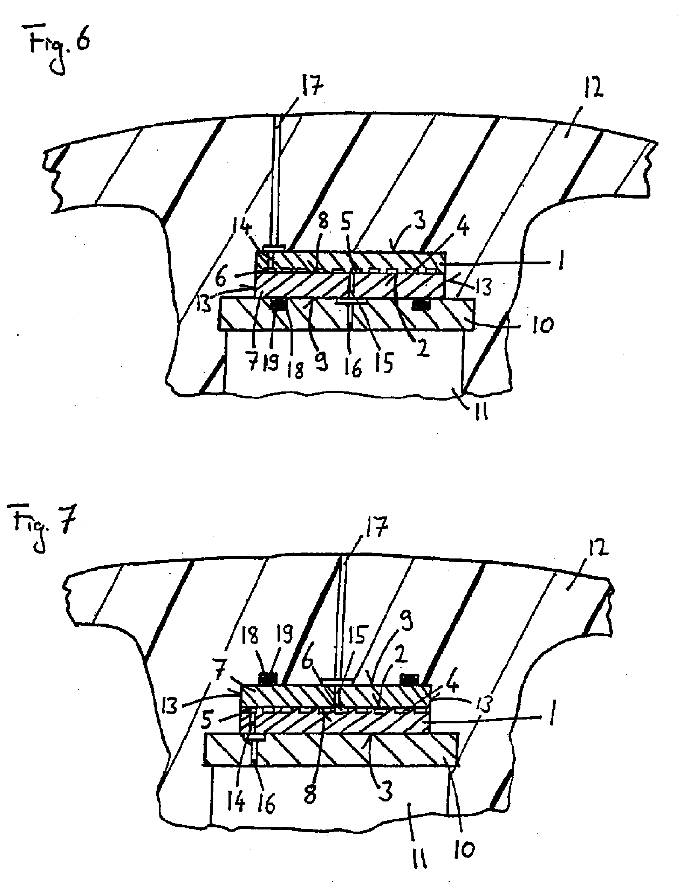

Figur 6 zeigt schematisch einen Querschnitt durch eine Infusionspumpe gemäß einem ersten Ausführungsbeispiel der Erfindung. Die Kanalplatte 1 ist zusammen mit einer Deckelplatte 7 benachbart zu einem Verschluss 10 eines Medikamentenraums 11 der Infusionspumpe angeordnet. Zusammen sind sie in ein Gehäuse 12 der Infusionspumpe eingebettet.

In dem Ausführungsbeispiel gemäß Figur 6 ist die Deckelplatte 7 aus Glas ausgebildet und so auf die Kanalplatte 1 aufgebondet, dass eine erste Seite 8 der Deckelplatte 7 an der Oberseite 2 der Kanalplatte 1 anliegt. Die erste Seite 8 der Deckelplatte 7 bildet somit die fehlende vierte Wand des Kanals 4. In diesem Ausführungsbeispiel ist die Deckelplatte 7 sandwichartig zwischen der Kanalplatte 1 und dem Verschluss 10 des Medikamentenraums 11 angeordnet.

Ein Einlass 16 in dem Verschluss 10 fluchtet mit einem Durchlass 15 durch die Deckelplatte 7. Durch den Einlass 16 und den Durchlass 15 durch die Deckelplatte 7 ist ein Fließweg für ein Medikament aus dem Medikamentenraum 11 zum Beginn 5 des Kanals 4 gebildet. Ein im Medikamentenraum 11 vorgehaltenes Medikament tritt beispielsweise aufgrund des Druckes eines verdampfenden Treibmittels durch diesen Fließweg in den Kanal 4 ein. Der Kanal 4 fungiert als Drosselstrecke für die Infusionspumpe und sein Fließwiderstand, der maßgeblich durch seinen Querschnitt und seine Länge bestimmt wird, dosiert die Abgabe des Medikaments.

Das Medikament durchfließt den Kanal 4 und tritt an dessen Ende 6 durch einen Durchlass 14 durch die Kanalplatte 1 und einen Auslass 17 in dem Gehäuse 12 aus, von wo aus es an seinen Wirkort in den Körper eines Patienten gelangt.

Die Deckelplatte 7 ist gegen den Verschluss 10 durch eine Dichtung aus einem O-Ring 19 abgedichtet. Dazu ist in dem Verschluss 10 eine ringförmige Ausnehmung 18 vorgesehen, in der der O-Ring 19 aus einem gummielastische Material angeordnet werden kann. Ähnliche Dichtungen können bei Bedarf auch zwischen anderen Teilen der Infusionspumpe vorgesehen sein.

Figur 7 zeigt ein alternatives Ausführungsbeispiel der Infusionspumpe. Bei diesem Ausführungsbeispiel sind Verschluss 10, Kanalplatte 1 und Deckelplatte 7 in einer anderen Reihenfolge gestapelt und die Kanalplatte 1 ist sandwichartig zwischen der Deckelplatte 7 und dem Verschluss 10 zum Medikamentenraum 11 angeordnet.

Bei diesem Ausführungsbeispiel liegt die Unterseite 3 der Kanalplatte an dem Verschluss 10 an. Die Oberseite 2 der Kanalplatte 1 wird durch die erste Seite 8 der Deckelplatte 7 abgedeckt. Auch in diesem Ausführungsbeispiel weist die Kanalplatte 1 einen Durchlass 14 auf, der hier jedoch mit dem Beginn 5 des Kanals 4 fluchtet, der im Randbereich der Kanalplatte 1 angeordnet ist. Das Ende 6 des Kanals 4 fluchtet mit einem Durchlass 15 durch die Deckelplatte 7 und einem Auslass 17 in den Gehäuse.

Bei diesem Ausführungsbeispiel ist die Deckelplatte 7 gegen des Gehäuse 12 durch einen O-Ring 19 abgedichtet, wobei der O-Ring 19 in dafür vorgesehenen Ausnehmungen 18 in dem Gehäuse angeordnet ist.

Die Erfindung sieht auch die folgenden durchnummerierten Ausführungsbeispiele vor. Rückbezüge deuten an, dass der Gegenstand als eine Unterkombination der zuvor genannten Gegenstand oder Gegenstände ausgebildet werden kann.
1. Infusionspumpe mit
   - einem Gehäuse (12) und einem innerhalb des Gehäuses (12) angeordneten Medikamentenraum (11) zur Aufnahme eines Medikaments;
   - einer die Abgabe des Medikamentes dosierenden, zu einem im Körper des Patienten mündenden Katheter führenden Drosselstrecke,
   wobei die Drosselstrecke als in eine Oberseite (2) einer Kanalplatte (1) eingebrachter Kanal (4) ausgebildet ist,
   dadurch gekennzeichnet, dass
   Bereiche (27) der Kanalplatte (1) ein mikrostrukturierbares Material aufweisen und Oberflächen (25, 26) des Kanals (4), die mit dem Medikament in Kontakt treten, ein gegenüber Lösungen mit einem pH-Wert von pH ≥ 10 resistentes Material aufweisen.
2. Infusionspumpe nach Ziffer 1,
   dadurch gekennzeichnet, dass
   als mikrostrukturierbares Material ein fotoempfindliches Glas vorgesehen ist.
3. Infusionspumpe nach Ziffer 1 oder 2,
   dadurch gekennzeichnet, dass
   als gegenüber basischen Lösungen mit einem pH-Wert von pH ≥ 10 resistentes Material ein fotoempfindliches Glas vorgesehen ist.
4. Infusionspumpe nach einem der Ziffern 1 bis 3,
   dadurch gekennzeichnet, dass
   die Kanalplatte (1) vollständig aus fotoempfindlichem Glas ausgebildet ist.
5. Infusionspumpe nach Ziffer 1,
   dadurch gekennzeichnet, dass
   als mikrostrukturierbares Material Silizium oder eine Siliziumverbindung vorgesehen ist.
6. Infusionspumpe nach Ziffer 5,
   dadurch gekennzeichnet, dass
   als gegenüber basischen Lösungen mit einem pH-Wert von pH ≥ 10 resistentes Material Glas vorgesehen ist.
7. Infusionspumpe nach Ziffer 6,
   dadurch gekennzeichnet, dass
   als gegenüber basischen Lösungen mit einem pH-Wert von pH ≥ 10 resistentes Material Borsilikatglas vorgesehen ist.
8. Infusionspumpe nach Ziffer 6,
   dadurch gekennzeichnet, dass
   als gegenüber basischen Lösungen mit einem pH-Wert von pH ≥ 10 resistentes Material Quarzglas vorgesehen ist.
9. Infusionspumpe nach Ziffer 6,
   dadurch gekennzeichnet, dass
   als gegenüber basischen Lösungen mit einem pH-Wert von pH ≥ 10 resistentes Material Kalknatronglas vorgesehen ist.
10. Infusionspumpe nach Ziffer 5,
   dadurch gekennzeichnet, dass
   als gegenüber basischen Lösungen mit einem pH-Wert von pH ≥ 10 resistentes Material ein Kunststoff vorgesehen ist.
11. Infusionspumpe nach Ziffer 10,
   dadurch gekennzeichnet, dass
   als gegenüber basischen Lösungen mit einem pH-Wert von pH ≥ 10 resistentes Material PTFE vorgesehen ist.
12. Infusionspumpe nach Ziffer 10,
   dadurch gekennzeichnet, dass
   als gegenüber basischen Lösungen mit einem pH-Wert von pH ≥ 10 resistentes Material DLC vorgesehen ist.
13. Infusionspumpe nach Ziffer 10,
   dadurch gekennzeichnet, dass
   als gegenüber basischen Lösungen mit einem pH-Wert von pH ≥ 10 resistentes Material ein Kunststoff aus der Gruppe bestehend aus Polyimid, Polyamid, Polyethylen, EVA, Polypropylen, Polymethylmethacrylat und PVC vorgesehen ist.
14. Infusionspumpe nach einem der Ziffern 1 bis 13,
   dadurch gekennzeichnet, dass
   der Kanal (4) eine Spiralform aufweist.
15. Infusionspumpe nach einem der Ziffern 1 bis 14,
   dadurch gekennzeichnet, dass
   der Kanal (4) einen rechteckigen Querschnitt aufweist.
16. Infusionspumpe nach einem der Ziffern 1 bis 15,
   dadurch gekennzeichnet, dass
   die Infusionspumpe eine Deckelplatte (7) mit einer ersten Seite (8) und einer zweiten Seite (9) aufweist, wobei die erste Seite (8) der Deckelplatte (7) an der Oberseite (2) der Kanalplatte (1) anliegt.
17. Infusionspumpe nach Ziffer 16,
   dadurch gekennzeichnet, dass
   die Deckelplatte (7) aus Glas ausgebildet ist.
18. Infusionspumpe nach Ziffer 16 oder 17,
   dadurch gekennzeichnet, dass
   durch einen Einlass (16) in einem Verschluss (10) des Medikamentenraums (11), durch einen Durchlass (15) durch die Deckelplatte (7), durch einen Durchlass (14) durch die Kanalplatte (1) und durch den Kanal (4) ein Fließweg für das Medikament gebildet ist.
19. Infusionspumpe nach Ziffer 18,
   dadurch gekennzeichnet, dass
   der Kanal (4) einen Beginn (5) und ein Ende (6) aufweist, wobei der Beginn (5) des Kanals (4) in einer Fließrichtung des Medikaments vor dem Ende (6) des Kanals (4) liegt.
20. Infusionspumpe nach Ziffer 19,
   dadurch gekennzeichnet, dass
   die Kanalplatte (1) mit ihrer Unterseite (3) und ihren Randseiten (13) in das Gehäuse (12) eingebettet ist und die Deckelplatte (7) zwischen der Kanalplatte (1) und einem Verschluss (10) des Medikamentenraums (11) angeordnet ist, wobei der Beginn (5) des Kanals (4) mit dem Durchlass (15) durch die Deckelplatte (7) und dem Einlass (16) in dem Verschluss (10) des Medikamentenraums (11) fluchtet und das Ende (6) des Kanals (5) mit dem Durchlass (14) durch die Kanalplatte (1) und einem Auslass (17) in dem Gehäuse (12) fluchtet.
21. Infusionspumpe nach Ziffer 20,
   dadurch gekennzeichnet, dass
   die zweite Seite (9) der Deckelplatte (7) gegen den Verschluss (10) des Medikamentenraums (11) mittels eines O-Ringes (19) aus einem gummielastischen Material abgedichtet ist.
22. Infusionspumpe nach Ziffer 21,
   dadurch gekennzeichnet, dass
   in dem Verschluss (10) des Medikamentenraums (11) Ausnehmungen (18) zur Aufnahme des O-Rings (19) angeordnet sind.
23. Infusionspumpe nach Ziffer 22,
   dadurch gekennzeichnet, dass
   die Deckelplatte (7) mit ihrer zweiten Seite (9) und ihren Randseiten (13) in das Gehäuse (12) eingebettet ist und die Kanalplatte (1) zwischen der Deckelplatte (7) und einem Verschluss (10) des Medikamentenraums (11) angeordnet ist, wobei der Beginn (5) des Kanals (4) mit dem Durchlass (14) durch die Kanalplatte (1) und dem Einlass (16) in dem Verschluss (10) des Medikamentenraums (11) fluchtet und das Ende (6) des Kanals (4) mit dem Durchlass (15) durch die Deckelplatte (7) und einem Auslass (17) in dem Gehäuse (12) fluchtet.
24. Infusionspumpe nach Ziffer 23,
   dadurch gekennzeichnet, dass
   die zweite Seite (9) der Deckelplatte (7) gegen das Gehäuse (12) mittels eines O-Ringes (19) aus einem gummielastischen Material abgedichtet ist.
25. Infusionspumpe nach Ziffer 24,
   dadurch gekennzeichnet, dass
   in dem Gehäuse (12) Ausnehmungen (18) zur Aufnahme des O-Rings (19) angeordnet sind.
26. Infusionspumpe nach einem der Ziffern 1 bis 25,
   dadurch gekennzeichnet, dass
   in dem Gehäuse (12) ein Treibmittelraum zur Aufnahme eines verdampfbarem Treibmittels angeordnet ist.
27. Infusionspumpe nach einem der Ziffern 1 bis 26,
   dadurch gekennzeichnet, dass
   die Infusionspumpe als in den Körper des Patienten implantierbare Infusionspumpe ausgeführt ist.
28. Kanalplatte (1) mit einer Drosselstrecke für eine Infusionspumpe, wobei die Drosselstrecke die Abgabe eines Medikamentes dosiert und zu einem im Körper des Patienten mündenden Katheter führt, wobei die Drosselstrecke als in eine Oberseite (2) der Kanalplatte (1) eingebrachter Kanal (4) ausgebildet ist,
   dadurch gekennzeichnet, dass
   Bereiche (27) der Kanalplatte (1) ein mikrostrukturierbares Material aufweisen und Oberflächen (25, 26) des Kanals (4), die mit dem Medikament in Kontakt treten, ein gegenüber basischen Lösungen mit einem pH-Wert von pH ≥ 10 resistentes Material aufweisen.
29. Kanalplatte nach Ziffer 28,
   dadurch gekennzeichnet, dass
   als mikrostrukturierbares Material ein fotoempfindliches Glas vorgesehen ist.
30. Kanalplatte nach Ziffer 28 oder 29,
   dadurch gekennzeichnet, dass
   als gegenüber basischen Lösungen mit einem pH-Wert von pH ≥ 10 resistentes Material ein fotoempfindliches Glas vorgesehen ist.
31. Kanalplatte nach einem der Ziffern 28 bis 30,
   dadurch gekennzeichnet, dass
   die Kanalplatte (1) vollständig aus fotoempfindlichem Glas ausgebildet ist.
32. Kanalplatte nach Ziffer 28,
   dadurch gekennzeichnet, dass
   als mikrostrukturierbares Material Silizium oder eine Siliziumverbindung vorgesehen ist.
33. Kanalplatte nach Ziffer 32,
   dadurch gekennzeichnet, dass
   als gegenüber basischen Lösungen mit einem pH-Wert von pH ≥ 10 resistentes Material Glas vorgesehen ist.
34. Kanalplatte nach Ziffer 33,
   dadurch gekennzeichnet, dass
   als gegenüber basischen Lösungen mit einem pH-Wert von pH ≥ 10 resistentes Material Borsilikatglas vorgesehen ist.
35. Kanalplatte nach Ziffer 33,
   dadurch gekennzeichnet, dass
   als gegenüber basischen Lösungen mit einem pH-Wert von pH ≥ 10 resistentes Material Quarzglas vorgesehen ist.
36. Kanalplatte nach Ziffer 33,
   dadurch gekennzeichnet, dass
   als gegenüber basischen Lösungen mit einem pH-Wert von pH ≥ 10 resistentes Material Kalknatronglas vorgesehen ist.
37. Kanalplatte nach Ziffer 32,
   dadurch gekennzeichnet, dass
   als gegenüber basischen Lösungen mit einem pH-Wert von pH≥ 10 resistentes Material ein Kunststoff vorgesehen ist.
38. Kanalplatte nach Ziffer 37,
   dadurch gekennzeichnet, dass
   als gegenüber basischen Lösungen mit einem pH-Wert von pH ≥ 10 resistentes Material PTFE vorgesehen ist.
39. Kanalplatte nach Ziffer 37,
   dadurch gekennzeichnet, dass
   als gegenüber basischen Lösungen mit einem pH-Wert von pH≥ 10 resistentes Material DLC vorgesehen ist.
40. Kanalplatte nach Ziffer 37,
   dadurch gekennzeichnet, dass
   als gegenüber basischen Lösungen mit einem pH-Wert von pH ≥ 10 resistentes Material ein Kunststoff aus der Gruppe bestehend aus Polyimid, Polyamid, Polyethylen, EVA, Polypropylen, Polymethylmethacrylat und PVC vorgesehen ist.
41. Kanalplatte nach einem der Ziffern 28 bis 40,
   dadurch gekennzeichnet, dass
   der Kanal (4) eine Spiralform aufweist.
42. Kanalplatte nach einem der Ziffern 28 bis 41,
   dadurch gekennzeichnet, dass
   der Kanal (4) einen rechteckigen Querschnitt aufweist.
43. Kanalplatte nach einem der Ziffern 28 bis 42,
   dadurch gekennzeichnet, dass
   der Kanal (4) einen Beginn (5) und ein Ende (6) aufweist, wobei der Beginn (5) des Kanals (4) in Fließrichtung des Medikaments vor dem Ende (6) des Kanals (4) liegt.
44. Kanalplatte nach Ziffer 43,
   dadurch gekennzeichnet, dass
   der Beginn (5) des Kanals (4) mit einen Durchlass (14) durch die Kanalplatte (1) fluchtet.
45. Kanalplatte nach Ziffer 43,
   dadurch gekennzeichnet, dass
   das Ende (6) des Kanals (4) mit einen Durchlass (14) durch die Kanalplatte (1) fluchtet.
46. Verfahren zur Herstellung einer Kanalplatte (1) mit einer Drosselstrecke für eine Infusionspumpe, wobei das Verfahren folgende Schritte aufweist:
   - Bereitstellen einer Kanalplatte (1) mit einer Oberseite (2) und einer Unterseite (3), wobei zumindest die Oberseite (2) fotoempfindliches Glas aufweist;
   - Belichten von Bereichen der Oberseite (2) der Kanalplatte (1) mit Licht einer Wellenlänge λg, für die das Glas fotoempfindlich ist,
   - Wärmebehandeln der Kanalplatte (1) durch Erhitzen auf eine Temperatur T_{G}, bei der in den belichteten Bereichen (23) eine Umwandlung der amorphen Glasstruktur in eine Kristallstruktur erfolgt;
   - Ätzen eines eine Drosselstrecke ausbildenden Kanals (4) in die Oberseite (2) der Kanalplatte (1) unter Inkontaktbringen der Kanalplatte (1) mit einer Ätzmittellösung und unter Entfernen der kristallinen Bereiche.
47. Verfahren nach Ziffer 46,
   wobei vor dem Schritt des Belichtens von Bereichen der Oberseite (2) der Kanalplatte (1) das Bereitstellen einer Fotolithografiemaske (20) zum Aufbringen eines Kanalmusters auf die Oberseite (2) der Kanalplatte (1) erfolgt.
48. Verfahren nach Ziffer 46,
   wobei das Belichten von Bereichen der Oberseite (2) der Kanalplatte (1) durch direktes Einbringen eines Kanalmusters in die Oberseite (2) der Kanalplatte (1) mittels eines Lasers erfolgt.
49. Verfahren einem der Ziffern 46 bis 48,
   wobei das Belichten der Oberseite (2) der Kanalplatte (1) mit Licht der Wellenlänge 290≤ λ_{G}≤ 330 nm erfolgt.
50. Verfahren nach einem der Ziffern 46 bis 49,
   wobei die Kanalplatte (1) zur Wärmebehandlung auf die Temperatur 500 °C ≤ T_{G} ≤ 600 °C erhitzt wird.
51. Verfahren nach einem der Ziffern 46 bis 50,
   wobei als Ätzmittellösung eine 10%ige Flusssäurelösung verwendet wird.
52. Verfahren nach einem der Ziffern 46 bis 51,
   wobei das Ätzen bei Raumtemperatur durchgeführt wird.
53. Verfahren zur Herstellung einer Kanalplatte (1) mit einer Drosselstrecke für eine Infusionspumpe, wobei das Verfahren folgende Schritte aufweist:
   - Bereitstellen einer Kanalplatte (1) mit einer Oberseite (2) und einer Unterseite (3), wobei zumindest die Oberseite (2) Bereiche (27) eines mikrostrukturierbaren Materials aufweist;
   - Bereitstellen einer Fotolithografiemaske (20) zum Aufbringen eines Kanalmusters auf die Oberseite (2) der Kanalplatte (1);
   - Aufbringen eines Fotolacks auf die Oberseite (2) der Kanalplatte (1);
   - Belichten der Oberseite (2) der Kanalplatte (1) durch die Fotolithografiemaske (20);
   - Ätzen eines eine Drosselstrecke ausbildenden Kanals (4) in die Oberseite (2) der Kanalplatte (1) unter Inkontaktbringen der Kanalplatte (1) mit einer Ätzmittellösung;
   - Beschichten von Oberflächen (25, 26) des Kanals (4), die mit einem Medikament in Kontakt treten, mit einem gegenüber basischen Lösungen mit einem pH-Wert von pH ≥ 10 resistenten Material.
54. Verfahren nach Ziffer 53,
   wobei als mikrostrukturierbares Material Silizium oder eine Siliziumverbindung verwendet wird.
55. Verfahren nach Ziffer 53 oder 54,
   wobei als gegenüber basischen Lösungen mit einem pH-Wert von pH≥ 10 resistentes Material Glas verwendet wird.
56. Verfahren nach Ziffer 55,
   wobei als gegenüber basischen Lösungen mit einem pH-Wert von pH ≥ 10 resistentes Material Borsilikatglas verwendet wird.
57. Verfahren nach Ziffer 55,
   wobei als gegenüber basischen Lösungen mit einem pH-Wert von pH ≥ 10 resistentes Material Quarzglas verwendet wird.
58. Verfahren nach Ziffer 55,
   wobei als gegenüber basischen Lösungen mit einem pH-Wert von pH≥ 10 resistentes Material Kalknatronglas verwendet wird.
59. Verfahren nach Ziffer 53 oder 54,
   wobei als gegenüber basischen Lösungen mit einem pH-Wert von pH≥ 10 resistentes Material ein Kunststoff verwendet wird.
60. Verfahren nach Ziffer 59,
   wobei als gegenüber basischen Lösungen mit einem pH-Wert von pH ≥ 10 resistentes Material PTFE verwendet wird.
61. Verfahren nach Ziffer 59,
   wobei als gegenüber basischen Lösungen mit einem pH-Wert von pH ≥ 10 resistentes Material DLC verwendet wird.
62. Verfahren nach Ziffer 59,
   wobei als gegenüber basischen Lösungen mit einem pH-Wert von pH ≥ 10 resistentes Material ein Kunststoff aus der Gruppe bestehend aus Polyimid, Polyamid, Polyethylen, EVA, Polypropylen, Polymethylmethacrylat und PVC verwendet wird.
63. Verfahren nach einem der Ziffern 59 bis 62,
   wobei das Beschichten der Oberflächen (25, 26) des Kanals (4) durch physikalische Gasphasenabscheidung erfolgt.
64. Verfahren nach einem der Ziffern 59 bis 62,
   wobei das Beschichten der Oberflächen (25, 26) des Kanals (4) durch chemische Gasphasenabscheidung erfolgt.
65. Verfahren zur Herstellung einer Infusionspumpe, das folgende Verfahrensschritte aufweist:
   - Herstellen einer Kanalplatte (1) nach einem der Ziffern 46 bis 64;
   - Einbringen zumindest eines Durchlasses (14) in die Kanalplatte (1), der mit einem Beginn (5) des Kanals (4) fluchtet;
   - Aufbonden einer Deckelplatte (7) aus Glas auf die Oberseite (2) der Kanalplatte (1), wobei ein Durchlass (15) in der Deckelplatte (7) mit dem Ende (6) des Kanals (4) fluchtet;
   - Einsetzen der Kanalplatte (1) mit aufgebondeter Deckelplatte (7) in ein Gehäuse (12).
66. Verfahren zur Herstellung einer Infusionspumpe, das folgende Verfahrensschritte aufweist:
   - Herstellen einer Kanalplatte (1) nach einem der Ziffern 46 bis 64;
   - Einbringen zumindest eines Durchlasses (14) in die Kanalplatte (1), der mit einem Ende (6) des Kanals (4) fluchtet;
   - Aufbonden einer Deckelplatte (7) aus Glas auf die Oberseite (2) der Kanalplatte (1), wobei ein Durchlass (15) in der Deckelplatte (7) mit dem Beginn (5) des Kanals (4) fluchtet;
   - Einsetzen der Kanalplatte (1) mit aufgebondeter Deckelplatte (7) in ein Gehäuse (12).

### Bezugszeichenliste

- 1: Kanalplatte
- 2: Oberseite
- 3: Unterseite
- 4: Kanal
- 5: Beginn
- 6: Ende
- 7: Deckelplatte
- 8: erste Seite
- 9: zweite Seite
- 10: Verschluss
- 11: Medikamentenraum
- 12: Gehäuse
- 13: Randseite
- 14: Durchlass durch die Kanalplatte
- 15: Durchlass durch die Deckelplatte
- 16: Einlass
- 17: Auslass
- 18: Ausnehmung
- 19: O-Ring
- 20: Maske
- 21: Durchbruch
- 22: Pfeil
- 23: belichteter Bereich
- 24: Grabenstruktur
- 25: Wände
- 26: Boden
- 27: Bereiche aus einem mikrostrukturierbaren Material
- 28: Bereiche aus einem gegen Basen resistenten Material

- T: Tiefe

## Patentansprüche

1. Infusionspumpe mit
- einem Gehäuse (12) und einem innerhalb des Gehäuses (12) angeordneten Medikamentenraum (11) zur Aufnahme eines Medikaments;
- einer die Abgabe des Medikamentes dosierenden, zu einem im Körper des Patienten mündenden Katheter führenden Drosselstrecke,
wobei die Drosselstrecke als in eine Oberseite (2) einer Kanalplatte (1) eingebrachter Kanal (4) ausgebildet ist,
**dadurch gekennzeichnet, dass**
Bereiche (27) der Kanalplatte (1) ein mikrostrukturierbares Material aufweisen und Oberflächen (25, 26) des Kanals (4), die mit dem Medikament in Kontakt treten, ein gegenüber Lösungen mit einem pH-Wert von pH≥ 10 resistentes Material aufweisen.

2. Infusionspumpe nach Anspruch 1,
**dadurch gekennzeichnet, dass**
als mikrostrukturierbares Material ein fotoempfindliches Glas vorgesehen ist.

3. Infusionspumpe nach Anspruch 1 oder 2,
**dadurch gekennzeichnet, dass**
als gegenüber basischen Lösungen mit einem pH-Wert von pH ≥ 10 resistentes Material ein fotoempfindliches Glas vorgesehen ist.

4. Infusionspumpe nach einem der Ansprüche 1 bis 3,
**dadurch gekennzeichnet, dass**
die Kanalplatte (1) vollständig aus fotoempfindlichem Glas ausgebildet ist.

5. Infusionspumpe nach Anspruch 1,
**dadurch gekennzeichnet, dass**
als mikrostrukturierbares Material Silizium oder eine Siliziumverbindung vorgesehen ist.

6. Infusionspumpe nach Anspruch 5,
**dadurch gekennzeichnet, dass**
als gegenüber basischen Lösungen mit einem pH-Wert von pH ≥ 10 resistentes Material Glas vorgesehen ist.

7. Infusionspumpe nach Anspruch 6,
**dadurch gekennzeichnet, dass**
als gegenüber basischen Lösungen mit einem pH-Wert von pH ≥ 10 resistentes Material eines der Gruppe bestehend aus Borsilikatglas, Quarzglas und Kalknatronglas vorgesehen ist.

8. Infusionspumpe nach Anspruch 5,
**dadurch gekennzeichnet, dass**
als gegenüber basischen Lösungen mit einem pH-Wert von pH ≥ 10 resistentes Material ein Kunststoff vorgesehen ist, wobei der Kunststoff eines der Gruppe bestehend aus PTFE, DLC, Polyimid, Polyamid, Polyethylen, EVA, Polypropylen, Polymethylmethacrylat und PVC vorgesehen ist.

9. Infusionspumpe nach einem der Ansprüche 1 bis 8,
**dadurch gekennzeichnet, dass**
der Kanal (4) eine Spiralform und/oder der Kanal einen rechteckigen Querschnitt aufweist.

10. Infusionspumpe nach einem der Ansprüche 1 bis 9,
**dadurch gekennzeichnet, dass**
die Infusionspumpe eine Deckelplatte (7) mit einer ersten Seite (8) und einer zweiten Seite (9) aufweist, wobei die erste Seite (8) der Deckelplatte (7) an der Oberseite (2) der Kanalplatte (1) anliegt.

11. Infusionspumpe nach Anspruch 10,
**dadurch gekennzeichnet, dass**
die Deckelplatte (7) aus Glas ausgebildet ist.

12. Infusionspumpe nach Anspruch 10 oder 11,
**dadurch gekennzeichnet, dass**
durch einen Einlass (16) in einem Verschluss (10) des Medikamentenraums (11), durch einen Durchlass (15) durch die Deckelplatte (7), durch einen Durchlass (14) durch die Kanalplatte (1) und durch den Kanal (4) ein Fließweg für das Medikament gebildet ist.

13. Infusionspumpe nach Anspruch 12,
**dadurch gekennzeichnet, dass**
der Kanal (4) einen Beginn (5) und ein Ende (6) aufweist, wobei der Beginn (5) des Kanals (4) in einer Fließrichtung des Medikaments vor dem Ende (6) des Kanals (4) liegt.

14. Infusionspumpe nach Anspruch 13,
**dadurch gekennzeichnet, dass**
die Kanalplatte (1) mit ihrer Unterseite (3) und ihren Randseiten (13) in das Gehäuse (12) eingebettet ist und die Deckelplatte (7) zwischen der Kanalplatte (1) und einem Verschluss (10) des Medikamentenraums (11) angeordnet ist, wobei der Beginn (5) des Kanals (4) mit dem Durchlass (15) durch die Deckelplatte (7) und dem Einlass (16) in dem Verschluss (10) des Medikamentenraums (11) fluchtet und das Ende (6) des Kanals (5) mit dem Durchlass (14) durch die Kanalplatte (1) und einem Auslass (17) in dem Gehäuse (12) fluchtet.

15. Infusionspumpe nach einem der Ansprüche 1 bis 14,
**dadurch gekennzeichnet, dass**
in dem Gehäuse (12) ein Treibmittelraum zur Aufnahme eines verdampfbarem Treibmittels angeordnet ist.
